# EUROPEAN PATENT APPLICATION

(11) **EP 2 085 906 A2**
(43) Date of publication of application: **05.08.2009**
(21) Application number: 08020532.1
(22) Date of filing: 26.11.2008
(51) Int. Cl.: G06F 19/00

(54) **Medical support control system**

(30) Priority: 29.01.2008 US 21897; 12.02.2008 US 29619
(71) Applicant: Olympus Medical Systems Corporation, Tokyo 151-0072 (JP)
(72) Inventor: Sekiguchi, Kiyoshi, Tokyo 151-0072 (JP); Ito, Masaru, Tokyo 151-0072 (JP); Tashiro, Koichi, Tokyo 151-0072 (JP); Furukawa, Nobuyuki, Tokyo 151-0072 (JP); Yamaki, Masahide, Tokyo 151-0072 (JP)
(74) Representative: von Hellfeld, Axel

(57) **Abstract**

A medical support control system comprising: a first controller (114,202) connected to at least one device; a second controller (202,114) connected to at least one device; a manipulation display device (221) shared by the first controller and the second controller, and alternately displaying a first graphical user interface (first GUI) created by the first controller and a second graphical user interface (second GUI) created by the second controller, wherein: each of the first GUI and the second GUI includes at least: a switching part (502-509, 511) for performing switching between the first GUI and the second GUI, and enabling control on a controller corresponding to a GUI after the switching; and an execution instruction part (521-525) for instructing the respective controllers to execute a process.

## Description

### Background of the Invention

### Field of the Invention

The present invention relates to a medical support control system for controlling medical devices and non-medical devices used for operations.

### Description of the Related Art

Operating systems using medical controllers or the like for controlling medical devices such as endoscopes or the like used for operations have been proposed. Medical devices to be controlled such as electric knives, insufflation devices, endoscope cameras, light source devices, or the like are connected to the medical controller (also referred to as an MC). Also, a display device, a manipulation panel, or the like is connected to the MC. The manipulation panel includes a display unit and a touch sensor, and is used as a central manipulation device by nurses, or the like working in an unsterilized area. The display device is used for displaying endoscope images or the like.

There is audio-visual equipment in the operating room, such as a room light, a room camera, an interphone device, a liquid crystal display device, or the like (non-medical devices). The audio-visual equipment is controlled independently or by a non-medical controller (also referred to as an NMC) used for the central control.

Japanese Patent Application Publication No. 2006-000536, for example, discloses an operating system, comprising:
a first controller connected to a medical device provided in an operating room;
a second controller connected to a non-medical device provided in the operating room; and
manipulation instruction input means transmitting the content of a manipulation instruction to the first controller when a manipulation instruction for the medical device or the non-medical device is input. The first controller transmits to the second controller a first control signal in accordance with the manipulation instruction of the non-medical device input into the manipulation instruction means. The second controller converts the first control signal into a second control signal used for controlling the non-medical device, and
transmits the second control signal to the non-medical device. Thereby, the operating system and a non-medical system work together, and the operating person himself/herself or the like can manipulate the non-medical devices.

### Summary of the Invention

A medical support control system according to the present invention comprises:
a first controller connected to at least one device;
a second controller connected to at least one device; and
a manipulation display device shared by the first controller and the second controller, and alternately displaying a first graphical user interface (first GUI) created by the first controller and a second graphical user interface (second GUI) created by the second controller, wherein:
   each of the first GUI and the second GUI includes at least:
      a switching part for performing switching between the first GUI and the second GUI, and enabling control on a controller corresponding to a GUI after the switching; and
      an execution instruction part for instructing the respective controllers to execute a process.

### Brief Description of the Drawings

Fig. 1 shows an entire configuration of the medical device control system according to the first embodiment;
Fig. 2 is a block diagram showing an entire configuration of a medical support control system 100 according to the first embodiment;
Fig. 3 is a block diagram showing a configuration of a NMC 202 according to the first embodiment;
Fig. 4A is a first view showing switching of a control target performed by a TP control switching unit 305 between the NMC 202 and the MC 114 according to the first embodiment;
Fig. 4B is a second view showing switching of a control target performed by the TP control switching unit 305 between the NMC 202 and the MC 114 according to the first embodiment;
Fig. 5 shows an example of an operation window according to the first embodiment;
Fig. 6 shows switching part information according to the first embodiment;
Fig. 7 is a flowchart for creating a window according to the first embodiment;
Fig. 8A is a first view showing switching of windows caused by switching of control between the MC 114 and the NMC 202 according to the first embodiment;
Fig. 8B is a second view showing switching of windows caused by switching of control between the MC 114 and the NMC 202 according to the first embodiment;
Fig. 9 shows an example of a category table according to the first embodiment;
Fig. 10 shows an example of transition of windows switched when category buttons or tabs are pressed according to the first embodiment;
Fig. 11 shows an operation window in which tabs are shared for prescribed devices according to the first embodiment;
Fig. 12 shows a labeling window for devices connected to a connection port of the NMC 202 according to the first embodiment;
Fig. 13 shows an operation window reformed on the basis of setting content of the labeling window according to the first embodiment; and
Fig. 14 is a flowchart for creating an operation window on the basis of performing setting in a labeling setting list according to the first embodiment.
Fig. 15A is a first figure showing a switching operation of a control target between the NMC 202 and an MC 114 performed by a TP control switching unit 305 according to the second embodiment;
Fig. 15B is a second figure showing the switching operation of a control target between the NMC 202 and the MC 114 performed by the TP control switching unit 305 according to the second embodiment; and
Fig. 16 shows interruption of communications, according to the second embodiment, through a TP coordinate communication line to the MC 114 and the NMC 202 in a case in which the TP image line is disconnected.

### Description of the Preferred Embodiments

### <First Embodiment>

Hereinafter, the first embodiment of the present invention will be explained in detail, referring to the drawings.

A medical support control system according to the present embodiment includes a medical device control system and a non-medical device control system. The medical device control system includes a plurality of medical devices and a medical controller for controlling these medical devices. The non-medical device control system includes non-medical devices (that may further include medical devices) that are used for operations, and a non-medical controller for controlling these non-medical devices.

An endoscopic operating system will be explained as an example of the medical device control system.

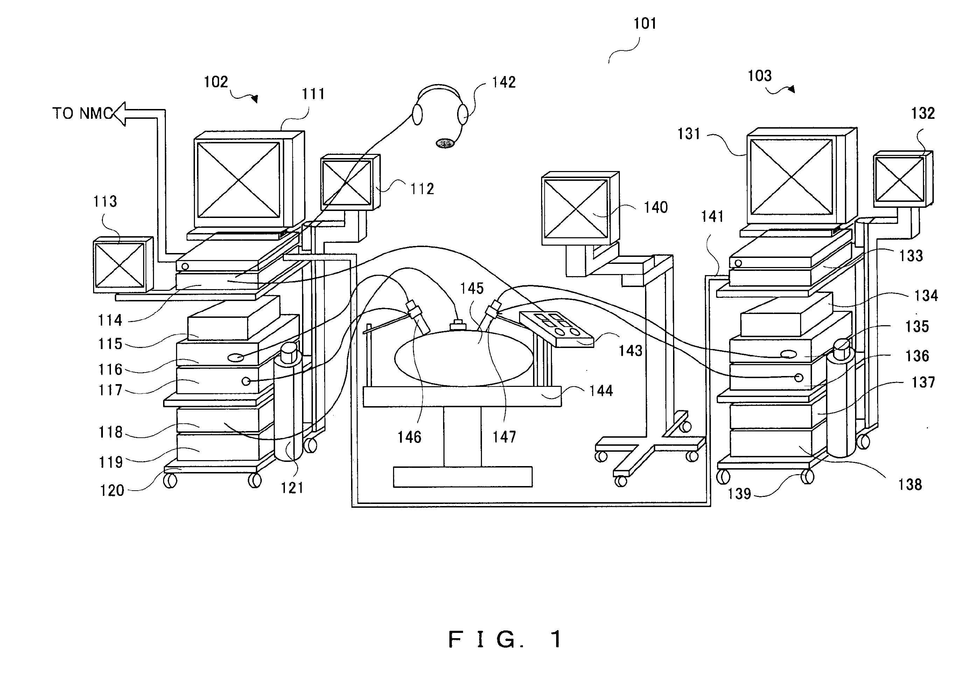

Fig. 1 shows an entire configuration of the medical device control systemaccording to the present embodiment. An endoscopic operating system is shown as a medical device control system 101. In the operating room, a first endoscopic operating system 102 and a second endoscopic operating system 103 beside a bed 144 on which a patient 145 is laid and a wireless remote controller 143 for the operating person are provided.

The endoscopic operating systems 102 and 103 respectively have first and second trolleys 120 and 139 each including a plurality of endoscope peripheral devices used for observation, examination, procedures, recoding, and the like. Also, an endoscope image display panel 140 is arranged on a movable stand.

On the first trolley 120, an endoscope image display panel 111, a central display panel 112, a central manipulation panel device 113, a medical controller (MC) 114, a recorder 115, a video processor 116, an endoscope light source device 117, an insufflation unit 118, and an electrical surgical device 119 are arranged.

The central manipulation panel device 113 is arranged in an unsterilized area to be used by nurses or the like in order to manipulate the respective medical devices in a centralized manner. This central manipulation panel device 113 may include a pointing device such as a mouse, a touch panel, or the like (not shown). By using the central manipulation panel device 113, the medical devices can be managed, controlled, and manipulated in a centralized manner.

The respective medical devices are connected to the MC 114 via communication cables (not shown) such as serial interface cables or the like, and can have communications with one another.

Also, a headset-type microphone 142 can be connected to the MC 114. The MC 114 can recognize voices input through the headset-type microphone 142, and can control the respective devices in accordance with the voices of the operating person.

The endoscope light source device 117 is connected to a first endoscope 146 through a light-guide cable used for transmitting the illumination light. The illumination light emitted from the endoscope light source device 117 is provided to the light guide of the first endoscope 146 and illuminates the affected areas or the like in the abdomen of the patient 145 into which the insertion unit of the first endoscope 146 has been inserted.

The optical image data obtained through the camera head of the first endoscope 146 is transmitted to a video processor 116 through a camera cable. The optical image data undergoes signal processing in a signal processing circuit in the video processor 116, and the video signals are created.

The insufflation unit 118 provides CO₂ gas to the abdomen of the patient 145 through a tube. The CO₂ gas is obtained from a gas tank 121.

On the second trolley 139, an endoscope image display panel 131, a central display panel 132, a expansion unit 133, a recorder 134, a video processor 135, an endoscope light source device 136, and other medical devices 137 and 138 (such as an ultrasonic processing device, a lithotripsy device, a pump, a shaver, and the like) are arranged. These respective devices are connected to the expansion unit 133 through cables (not shown) , and can communicate with one another. The MC 114 and the expansion unit 133 are connected to each other through the expansion cable 141.

The endoscope light source device 136 is connected to a second endoscope 147 through the light-guide cable for transmitting the illumination light. The illumination light emitted from the endoscope light source device 136 is provided to the light guide of the second endoscope 147, and illuminates the affected areas or the like in the abdomen of the patient 145 into which the insertion unit of the second endoscope 147 has been inserted.

The optical image data obtained through the camera head of the second endoscope 147 is transmitted to a video processor 135 through a camera cable. The optical image data undergoes signal processing in a signal processing circuit in the video processor 135, and the video signals are created. Then, the video signals are output to the endoscope image display panel 131, and endoscope images of the affected areas or the like are displayed on the endoscope image display panel 131.

Further, the MC 114 can be controlled by the operating person manipulating the devices in the unsterilized area. Also, the first and second trolleys 120 and 139 can include other devices such as printers, ultrasonic observation devices, or the like.

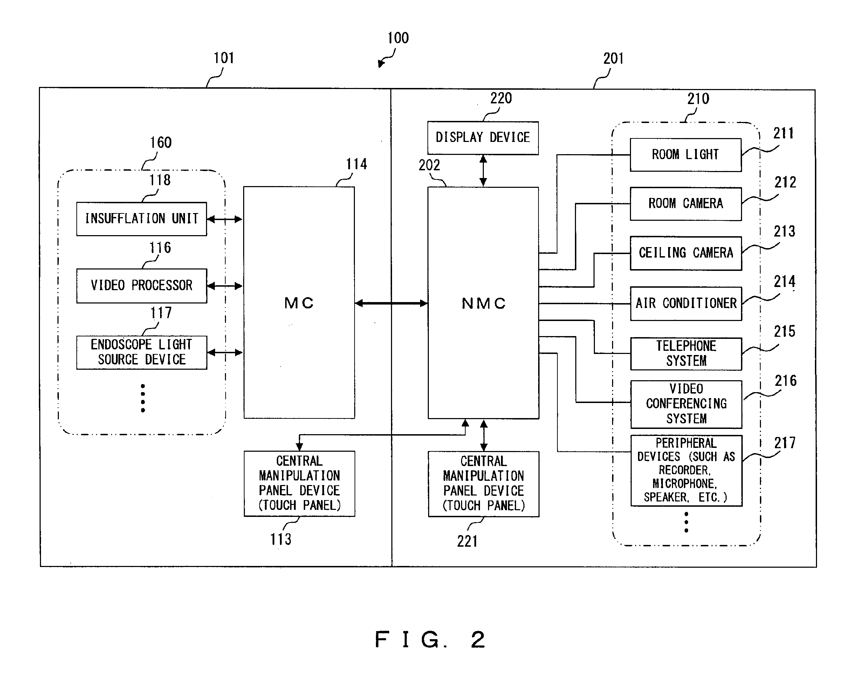

Fig. 2 is a block diagram showing an entire configuration of a medical support control system 100 according to the present embodiment. As described above, the medical support control system 100 includes the medical device control system 101 and a non-medical device control system 201. A detailed configuration of the medical device control system 101 is as shown in Fig. 1. However, in Fig. 2, the medical device control system 101 is shown in a simplified manner for simplicity of explanation.

In Fig. 2, a medical device group 160 is a group of medical devices that are directly connected to the medical controller 114 or are indirectly connected to the MC 114 via the expansion unit 133. Examples of the devices included in the medical device group 160 are the insufflation unit 118, the video processor 116, the endoscope light source device 117, the electrical surgical device 119, and the like.

The central manipulation panel device 113 has a touch panel, and in accordance with the information input into the touch panel, the devices connected to the MC 114 or a non-medical device controller (NMC) 202 that will be described later can be manipulated.

The non-medical control system 201 includes the NMC 202 connected to the MC 114 through a communication cable or the like, and a non-medical device group 210. In this configuration, the NMC 202 can transmit and receive, through an image cable, the video signals to and from the medical device group 160 connected to the MC 114.

The NMC 202 controls the non-medical devices (including the audio-visual devices) connected thereto. As shown in Fig. 2, the non-medical device group 210 connected to the NMC 202 according to the present embodiment consists of a room light 211, a room camera 212, a ceiling camera 213, an air conditioner 214, a telephone system 215, a conference system 216 to be used for individuals in remote places (referred to as a video conference system hereinafter), and other peripheral devices 217. Further, a display device 220 and a central manipulation panel device 221 are connected to the NMC 202.

Also, the non-medical device group 210 includes equipment such as light devices provided in the operating room in addition to the AV devices used for recording and reproducing image data.

The display device 220 is a plasma display panel (PDP) or a liquid crystal display (LCD) device, and displays images of the predetermined device or images of the devices selected by nurses or the like through the central manipulation panel device 221. The room light 211 is a device that illuminates the operating room. The room camera 212 is used for shooting images of the situations in the operating room. The ceiling camera 213 is a camera suspended from the ceiling, whose positions can be changed. The conference system 216 is a system that displays images and transmits voices of nurses or the like in the medical office or the nurse stations, and enables conversations with them. The peripheral devices 217 are, for example, a printer, a CD player, a DVD recorder, and the like. The central manipulation panel device 221 has a touch panel that is the same as that included in the central manipulation panel device 113, and controls the respective AV devices connected to the NMC 202. The central manipulation panel devices 113 and 221 are referred to as TPs hereinafter.

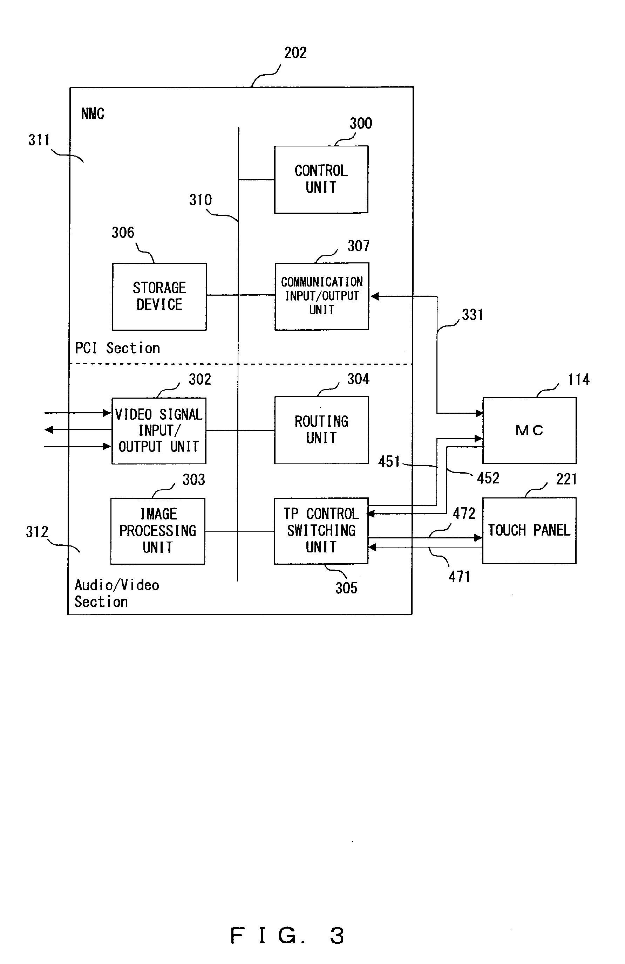

Fig. 3 is a block diagram showing a configuration of the NMC 202 in the present embodiment. The NMC 202 includes a PIC section 311 and an audio/video (A/V) section 312.

The PCI section 311 mainly controls a non-medical device group 210 connected to the NMC 202. The PCI section 311 includes a control unit 300, a storage device 306, and a communication input/output unit 307. Numeral 310 denotes a back plane.

The control unit 300 controls the entirety of the PCI section 311, and transmits and receives data to and from the A/V section 312. The control unit 300 creates Graphical User Interface image information (hereinafter referred to as GUI image information) that is an image layout to be displayed on a TP 221 or a monitor device, and transmits it to a routing unit 304.

The storage device 306 stores various programs, information set by the TP 221, and the like. The communication input/output unit 307 is a communication interface used for the communications with the MC 114 via a communication line 331.

The A/V section 312 is a section that mainly processes the video signals and the audio signals. The A/V section 312 includes a video signal input/output unit 302, an image processing unit 303, a routing unit 304, and a TP control switching unit 305.

The video signal input/output unit 302 has a plurality of video signal input ports and a plurality of video signal output ports.

The routing unit 304 switches routes for the video signals that were processed in the image processing unit 303 and for the video signals input from the video signal input/output unit 302, and transfers them to a prescribed configuration unit in the NMC 202. Also, the routing unit 304 transfers to the TP control switching unit 305 the GUI image information created in the control unit 300.

The image processing unit 303 performs image processing on the image information transferred from the routing unit 304. Examples of the image processing are the enlargement/reduction (scaling) of images, the mirroring of images, the rotation of images, displaying another, smaller image in a main image (picture in picture (PIP)), and displaying a plurality of images simultaneously (picture out picture (POP)).

TP coordinate communication lines 451 and 471 are communication lines through which TP coordinate signals generated by touch manipulations on the TP 221 are conveyed. TP image lines 452 and 472 are image lines through which image signals such as GUI images or the like to be displayed on the TP 221 are conveyed.

The TP control switching unit 305 synthesizes the GUI image created in the control unit 300 with images created on the basis of the video signals transmitted from the video signal input/output unit 302. Then, the TP control switching unit 305 outputs the synthesized image to the TP 221. Further, the TP control switching unit 305 can perform switching between the NMC 202 and the MC 114 as the manipulation targets of the TP 221. In other words, the TP control switching unit 305 receives TP coordinate information from the TP 221 in accordance with touch manipulation, transfers the received TP coordinate information to the control unit 300, or transfers the TP coordinate information to the MC 114.

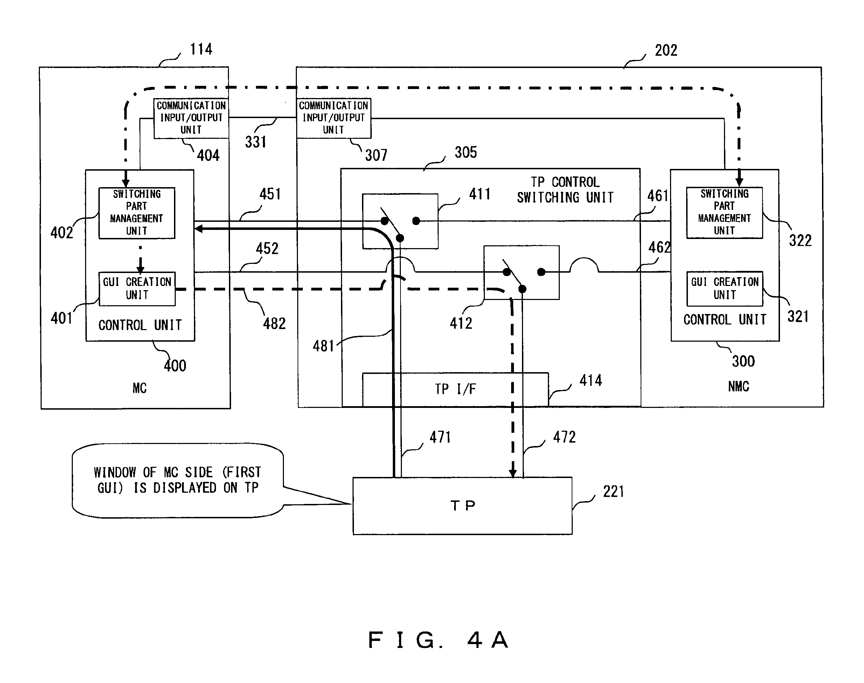

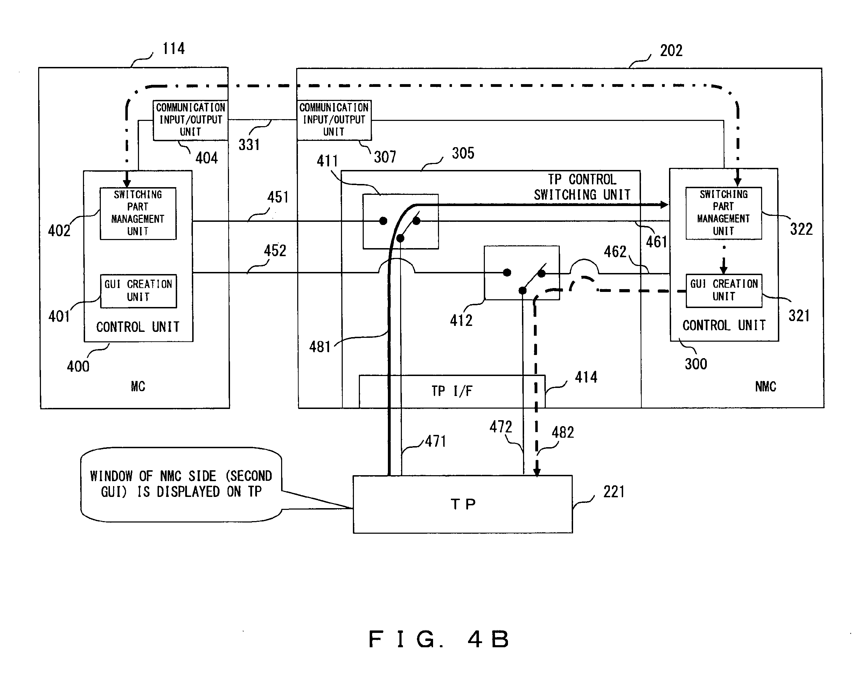

Figs. 4A and 4B respectively show the switching of the control targets between the NMC 202 and the MC 114 by using the TP control switching unit 305.

As described above, the MC 114 is a controller mainly for the medical devices. The MC 114 creates a graphical user interface (first GUI) that is a window used for controlling the medical devices belonging to the MC 114 itself.

As described above, the NMC 202 is a controller mainly for the non-medical devices. The NMC 202 creates a graphical user interface (second GUI) that is a window used for controlling the non-medical devices belonging to the NMC 202 itself.

The first and second GUIs are designed on the basis of a common graphical user interface (common GUI). For example, by switching the tabs on a window on the TP 221, the first and second GUIs can be switched therebetween.

The control units 300 and 400 have GUI creation units 321 and 401 and switching part management units 322 and 402. The word "part" refers to a constituent element of a window displayed on the TP 221. The word "switching part" refers to a category button or a tab displayed on a window of the TP 221. This will be described later.

The switching part management units 322 and 402 exchange with each other switching part information of the MC 114 side and switching part information of the NMC 202 side. The GUI creation unit 321 creates a second GUI on the basis of the switching part information provided from the switching part management unit 322. The GUI creation unit 401 creates the first GUI on the basis of the switching part information provided from the switching part management unit 402.

The TP coordinate communication line 451, 461, and 471 are communication lines for transmitting, to the control unit 300 or 400, the TP coordinate signals from the TP 221.

The TP image lines 452, 462, and 472 are image lines for transmitting, from the control unit 400 of the MC 114 to the TP 221 or from the control unit 300 of the NMC 202 to the TP 221, the video signals of the first GUI images or the second GUI images to be displayed on the TP 221.

The TP control switching unit 305 has a TP coordinate communication line switch 411, a TP image line switch 412, and a TP I/F 414.

The TP I/F 414 is an interface to which the TP image line and the TP coordinate communication line between the TP 221 and the NMC 202 are connected.

The TP coordinate communication line switch 411 is a switch that determines whether the TP coordinate communication line 471 is to be connected to the TP coordinate communication line 451 of the MC 114 side or the TP coordinate communication line 461 of the NMC 202 side.

The TP image line switch 412 is a switch that determines whether the TP image line 472 is to be connected to the TP image line 452 of the MC 114 side or the TP image line 462 of the NMC 202 side.

A communication input/output unit 404 is a communication interface used for performing communications with the NMC 202 via the communication line 331.

The control units 300 and 400 communicate with and monitor each other via the communication line 331. Also, the control unit 300 and the control unit 400 synchronize each other's GUI environment, and exchange information that has to be held by both of them for configuring a common GUI. An example of the above information is window elements (window element information such as tab names) to be used commonly.

Explanations will be given for a case shown in Fig. 4A in which the TP coordinate communication line 451 is selected by the switch 411 and the TP image line 452 is selected by the switch 412. The switching part management unit 402 provides to the GUI creation unit 401 the switching part information that was provided to the switching part management unit 322, and provides to the GUI creation unit 401 the switching part information provided from the switching part management unit 322. A TP image signal 482 created in the GUI creation unit 401 is input into the TP 221 via the TP image line switch 412, and the first GUI is displayed on the TP 221. In this case, a TP coordinate signal 481 created by touch manipulations on the first GUI displayed on the TP 221 is input into the control unit 400 via the switch 411 from the TP 221.

Next, explanations will be given for a case, shown in Fig. 4B, in which the TP coordinate communication line 461 is selected by the switch 411, and the TP image line 462 is selected by the switch 412. The switching part management unit 322 provides to the GUI creation unit 321 the switching part information that was provided to the switching part management unit 402, and also provides to the GUI creation unit 321 the switching part information that was provided from the switching part management unit 402. A TP image signal 482 created in the GUI creation unit 321 is input into the TP 221 via the switch 412, and the second GUI is displayed on the TP 221. A TP coordinate signal 481 created by touch manipulations on the second GUI displayed on the TP 221 is input into the control unit 300 via the switch 411 from the TP 221.

Next, explanations will be given for a case in which the first GUI transits to the second GUI in response to the touch manipulations on the TP 221. The TP coordinate signal 481 created by the touch manipulations on the TP 221 is sent to the control unit 400 in the MC 114. The control unit 400 that has received the TP coordinate signal 481 reports, to the control unit 300 in the NMC 202 via the communication line 331, that the first GUI will be switched to the second GUI. When receiving this report, the control unit 300 controls the TP control switching unit 305, and the switches 411 and 412 are operated so that the TP coordinate communication line 461 and the TP image line 462 on the NMC 202 side are enabled. Then, the control unit 300 causes the TP 221 to display the second GUI via the TP image lines 462 and 472. Additionally, this process is also applied to the case in which the second GUI transits to the first GUI in response to the touch manipulations.

As described above, the target to be controlled by the TP 221 is changed between the MC 114 and the NMC 202 on the basis of the switching operations of the TP control switching unit 305. This switching operation is not perceived by the users, and accordingly the users feel as if they have controlled only one controller.

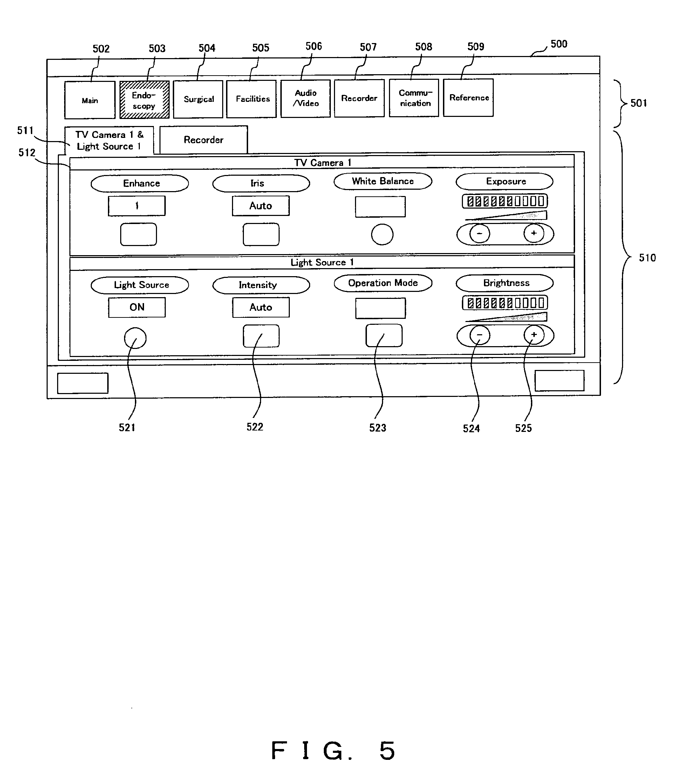

Fig. 5 shows an example of an operation window according to the present embodiment. Fig. 5 shows an example of the operation window displayed when the TV camera and the light source device are to be manipulated as the control target of the MC 114. An operation window 500 mainly includes a category button display area 501 and a tab-content display area 510.

Category buttons of "main window" 502, "Endoscopy" 503, "Surgical" 504, "Facilities" 505, "Audio/Video" 506, "Recorder" 507, "Communication" 508, and "Reference" 509 are displayed on the category button display area 501. When these buttons are pressed, information of the devices belonging to the respective categories are displayed on the tab-content display area 510. For example, when the category button "Endoscopy" 503 is pressed, the names of "TV camera 1 and Light source 1" and "Recorder" are displayed on tabs 511.

The tab-content display area 510 includes the tabs 511 and a selection-tab-content display unit 512. When one tab is selected, setting information of the device corresponding to the name of the selected tab, buttons 521-525 for operating control of devices, images picked up by the device, and the like are displayed on the selection-tab-content display unit 512. In Fig. 5, the tab "TV Camera 1 and Light Source 1" is selected, and thus setting items of "TV Camera 1" and "Light Source 1" are displayed on the selection-tab-content display unit 512.

As described above, the window displayed on the TP 221 has a plurality of parts such as the category buttons and the tab-content display area 510 (tabs, selection boxes, input boxes, buttons, images, label information, text information, and the like). As described above, the scope of the word "switching part" includes the category buttons (category selection part) and the tabs (tab selection part). Also, parts that are used for giving instructions for executing processes to the respective controllers (for example, buttons 521-525 for operating control of devices) are referred to as execution instruction parts.

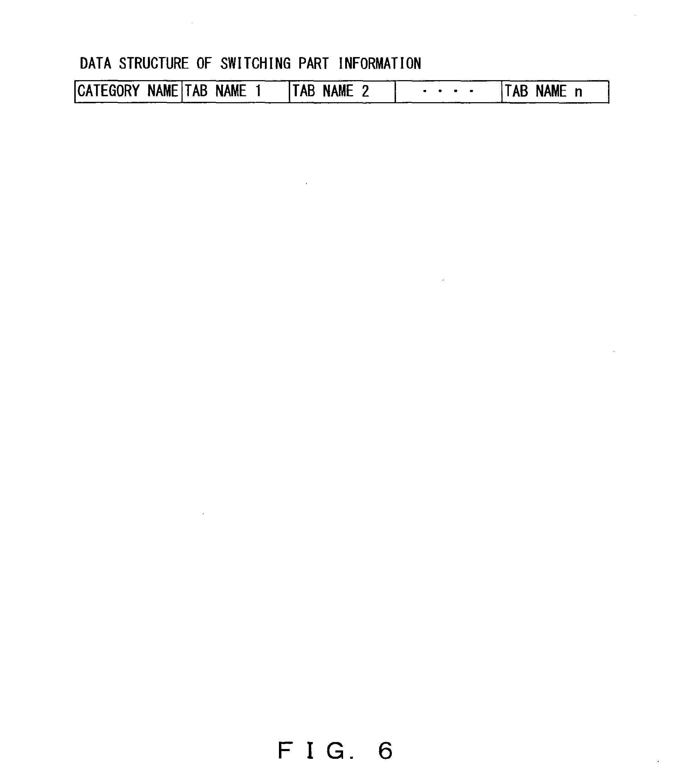

Fig. 6 shows switching part information according to the present embodiment. The switching part information includes a category name and at least one tab name (device name(s)) belonging to that category. The switching part management units 322 and 402 manage the devices to be controlled by the respective controllers as the switching part information, and exchange the switching part information with each other.

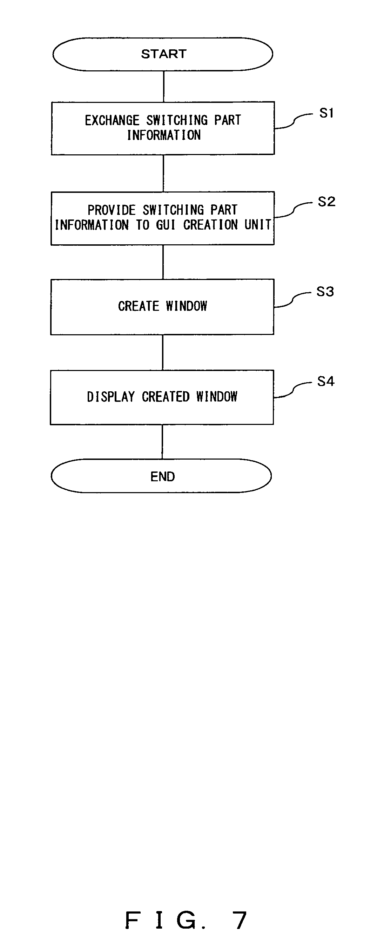

Fig. 7 is a flowchart for creatingwindows according to the present embodiment. Programs for the present embodiment are stored respectively in the storage devices in the MC 114 and the NMC 202. The control units 300 and 400 respectively read the programs from their own storage devices, and execute the processes described in the flowchart in Fig. 7. For simplicity of explanation, the constituent elements on the controller side that is the control target of the TP 221 are referred to as a control unit Ca, a switching part management unit C1a, and a GUI creation unit C2a, and the constituent elements on the other controller side are referred to as a control unit Cb, a switching part management unit C1b, and a CUI creation unit C2b.

Accordingly, in the case shown in Fig. 4A, the control unit 400, the switching part management unit 402, and the GUI creation unit 401 respectively correspond to Ca, C1a, and C2a. Also, the control unit 300, the switching part management unit 322, and the GUI creation unit 321 respectively correspond to Cb, C1b, and C2b.

Also, in the case shown in Fig. 4B, the control unit 300, the switching part management unit 322, and the GUI creation unit 321 respectively correspond to Ca, C1a, and C2a. Also, the control unit 400, the switching part management unit 402, and the GUI creation unit 401 respectively correspond to Cb, C1b, and C2b.

First, the switching part information is exchanged between the switching part management units C1a and C1b (S1). Thereafter, the switching part management unit C1a provides to the GUI creation unit C2a the switching part information that was provided to the switching part management unit C1b and also provides to the GUI creation unit C2a the switching part information that was provided from the switching part management unit C1b. The GUI creation unit C2a creates a window to be displayed on the TP 221 on the basis of the obtained switching part information (S3), and causes the TP 221 to display the window (S4).

Thereby, not only the tabs corresponding to the devices controlled by one controller, but also the tabs corresponding to the devices controlled by the other controller can be displayed on one and the same window.

As described above, it is possible to control the controllers as one controller without consciousness of two controllers. In other words, the two controllers share the switching part management information via communication lines and cause the shared switching parts to be displayed on a window, and if a tab of the counterpart side is selected, control is shifted to the counterpart. Thereby, a sense of discomfort caused when two controllers respectively have manipulation windows similar to each other and these windows have to be switched can be reduced.

There is a case in which the selection-tab-content display unit 512 after the switching is not ready to be displayed when the controllers are switched as shown in Figs. 4A and 4B due to the tabs being switched. In such a case an intermediate image is temporarily displayed before the selection-tab-content display unit 512 after the switching becomes ready to be displayed.

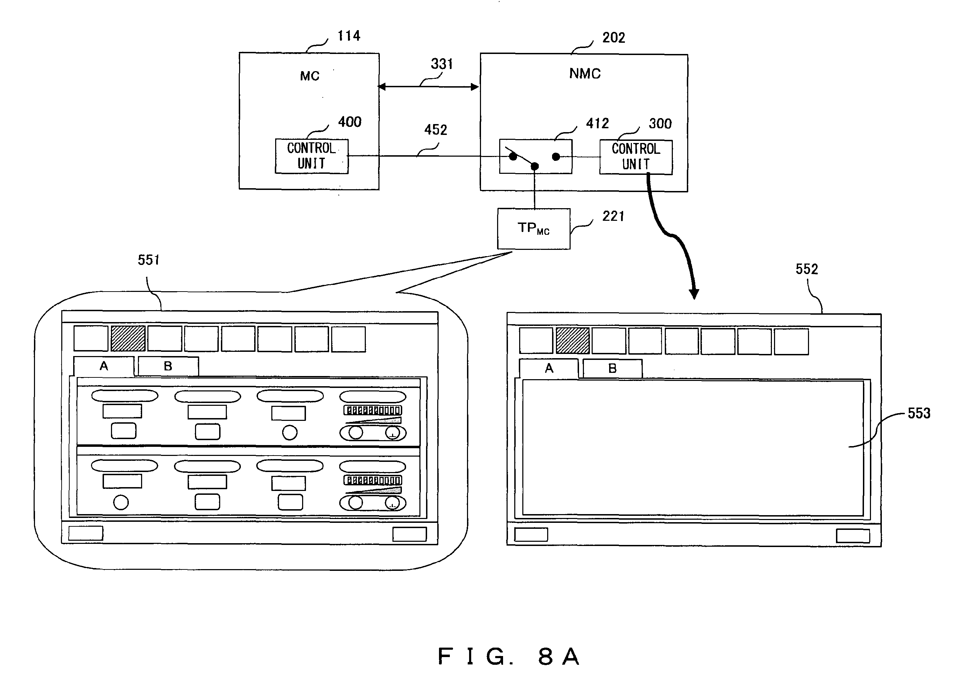

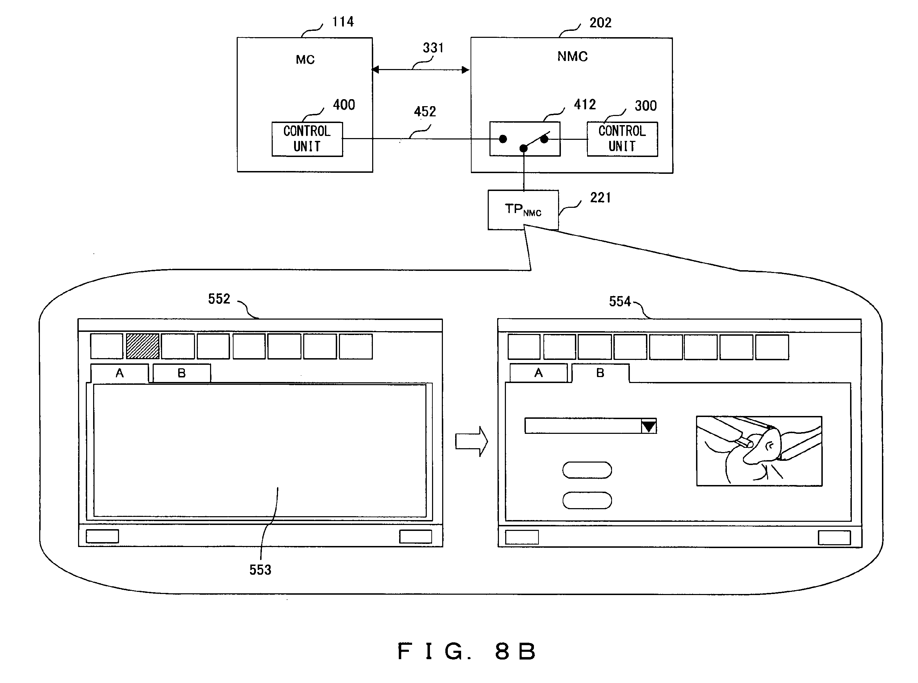

By referring to Figs. 8A and 8B, explanation will be given for the switching of windows caused by the switching of the control between the MC 114 and the NMC 202. When tab "A" in the window 551 is selected, the control target of the TP 221 is the MC 114. When tab "B" is selected, the control target of the TP 221 is the NMC 202.

Fig. 8A is a first view showing the switching of windows caused by the switching of the control between the MC 114 and the NMC 202 according to the present embodiment. In Fig. 8A, the control is shifted to the MC 114 side, and the window 551 created in the MC 114 is displayed on the TP 221. Then, the NMC 202 corresponds to the window 551 displayed by the MC 114, and a window 552 having a blank space as an intermediate image 553 in the selection tab-content display area of the tab selected in the window 551 is continuously held.

Fig. 8B is a second view showing the switching of windows caused by the switching of the control between the MC 114 and the NMC 202. Fig. 8B shows a transition of windows caused by the shift of control from the MC 114 to the NMC 202 due to switching of the tabs. In Fig. 8A, when tab "B" is selected, the control is shifted from the MC 114 to the NMC 202.

In this configuration, depending upon the communication environment, it sometimes happens that the timing of the switch 412 switching windows and the timing of creating the selection tab-content display area of the tab "B" that is to be displayed after the switching do not correspond to each other, and the selection tab-content display area does not appear smoothly. In order to cope with this, a window 552 having the intermediate image 553 is displayed temporarily on the TP 221 until the selection-tab-content display unit 512 after the switching of the tabs becomes ready to be displayed. When the selection-tab-content display unit 512 after the switching becomes ready, a window 554 is displayed. This is applied also to the transition of windows caused by the shift of the control from the NMC 202 to the MC 114. Thereby, manipulation does not cause a sense of discomfort even when the transition of windows caused by the switching of the controllers is realized.

Next, explanation will be given for the tab-content display area 510 that is switched when the category buttons are pressed. As described above, when a category button is newly pressed, the tab belonging to the selected category is displayed. When categories are switched, tab information for identifying the tab that had been selected immediately before the switching is stored in a category table. When that category is again selected, the selection states of the tabs are reproduced on the basis of the stored tab information.

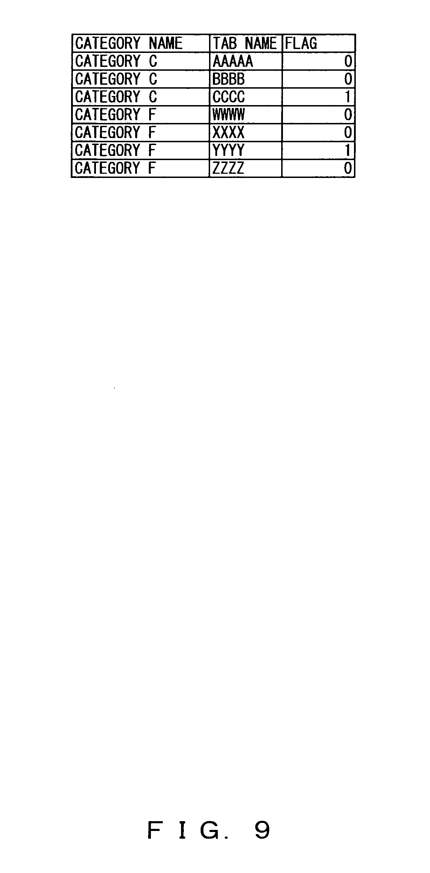

Fig. 9 shows an example of the category table according to the present embodiment. The category table is stored in the storage device in each controller. The category table includes data items of "category name", "tab name", and "flag". In Fig. 9, tabs "AAAA", "BBBB", and "CCCC" belong to "category C". Also, tabs "WWWW", "XXXX", "YYYY" and "ZZZZ" belong to "category F". The flag is used for identifying the tab that had been selected immediately before the switching of the tabs ("0" represents that the tab had not been selected, and "1" represents that the tab had been selected).

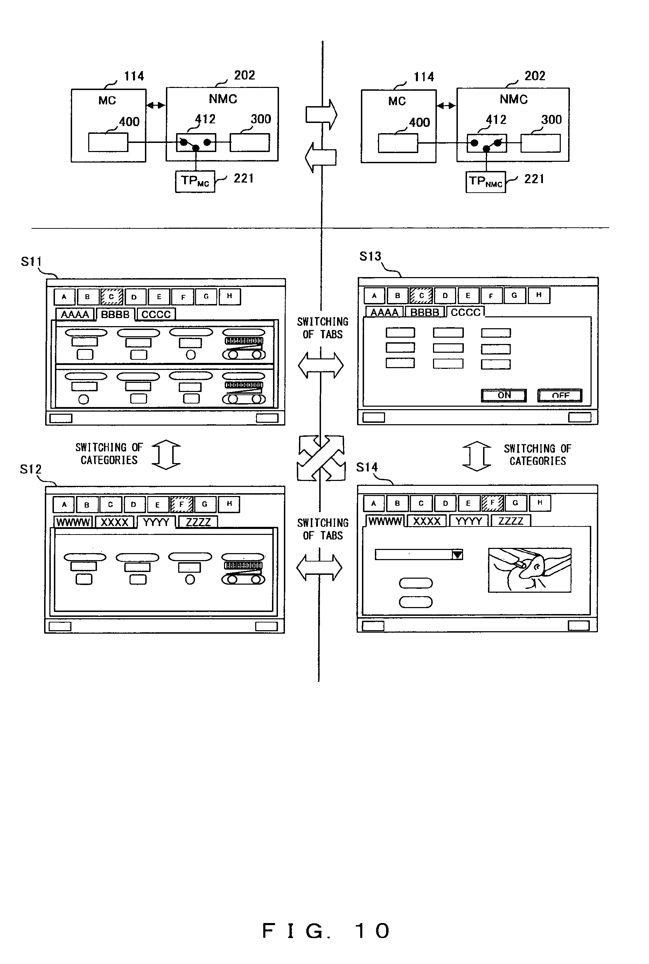

Fig. 10 shows an example according to the present embodiment of a transition of windows caused by the category buttons and tab buttons being pressed. The windows of S11 and S12 are windows controlled by the MC 114 side. The windows of S13 and S14 are windows controlled by the NMC 202 side. The relationships between S11 and S12, between S11 and S14, between S12 and S13, and between S13 and S14 represent switching of the categories. Relationships between S11 and S13 and between S12 and S14 represent switching of the tabs. Hereinafter, the relationship between S12 and S13 will be explained.

In S13, "category C" is selected, and the tabs "AAAA", "BBBB", and "CCCC" belonging to "category C" are displayed, and the tab "CCCC" is selected.

When "category F" is pressed in the state of S13, the control unit 300 updates the flags in the category table in the storage device 306 in order to identify the tab that is currently selected. In this case, the "flag" of the tab "CCCC" of "category C" is set to "1" in the category table. The update information of the category table is provided to the MC 114. Thereafter, the subject of control is switched to the MC 114.

Then, as shown in S12, tabs "WWWW", "XXXX", "YYYY", and "ZZZZ" belonging to "category F" are displayed on the tab-content display area 510. Further, the control unit 400 reads the category table in the storage device in the MC 114. The control unit 400 searches the table for the tab name whose flag is "1" from among the tab names belonging to "category F", and causes the tab "YYYY" to be selected.

This process is performed also when S12 transits to S13. Also, this process is performed when S13 transits to S14. Also, the category table is shared by the MC 114 and the NMC 202 via the communication line 331. Thereby, as indicated by the relationships between S11 and S14 and between S12 and S13, it is possible to respond to switching of categories occurring together with switching of the controllers.

By employing the above configuration, it is possible for the MC 114 and the NMC 202 to share the window information (tab information) that was last displayed in each category. Accordingly, when categories are switched by pressing category buttons, users feel as if they have manipulated the same controller window and do not feel a sense of discomfort even when the controllers are switched.

As described above, in an operation window, a tab is displayed for each function or each device (function selection tab or operation tab). By selecting tabs in accordance with usages or purposes, it is possible to use desired functions or to control desired devices. Tabs are displayed for each function; thus, the number of tabs increases as functions are added. Thereby, it is easy to understand which devices or which functions are available by viewing tabs displayed on windows. However, as the number of tabs increases, the actual manipulation areas have to be smaller, and the visibility decreases. In order to cope with this situation, when a plurality of the devices whose tab names are the same are to be displayed on an operation window, the tabs are shared, and manipulation items of such devices are displayed on the same tab in a merged manner.

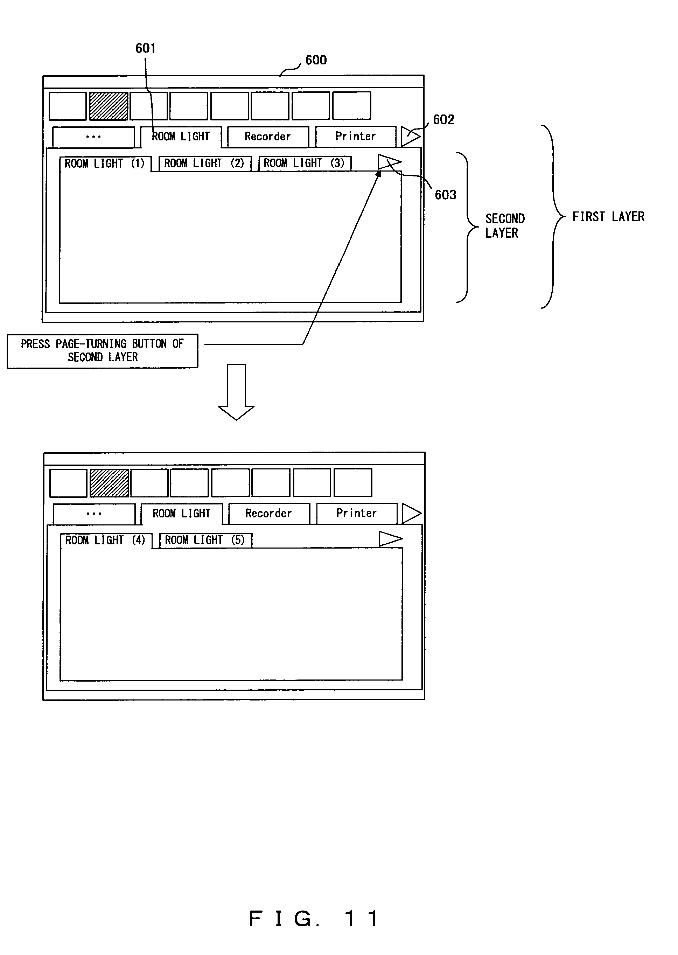

Fig. 11 shows an operation window in which tabs for prescribed devices are shared according to the present embodiment. When tabs of the same device names in the same device category are to be displayed, e.g., when a plurality of room lights are to be displayed, an operation window in which a plurality of tabs are included in the same tab is displayed. In Fig. 11, an operation window 600 in which second-layer tabs "room light" are included in a first-layer tab "room light" 601 is shown. When all the tabs cannot be included in the first layer or the second layer, the tabs displayed on the next page can be selected by pressing page-turning buttons 602 and 603.

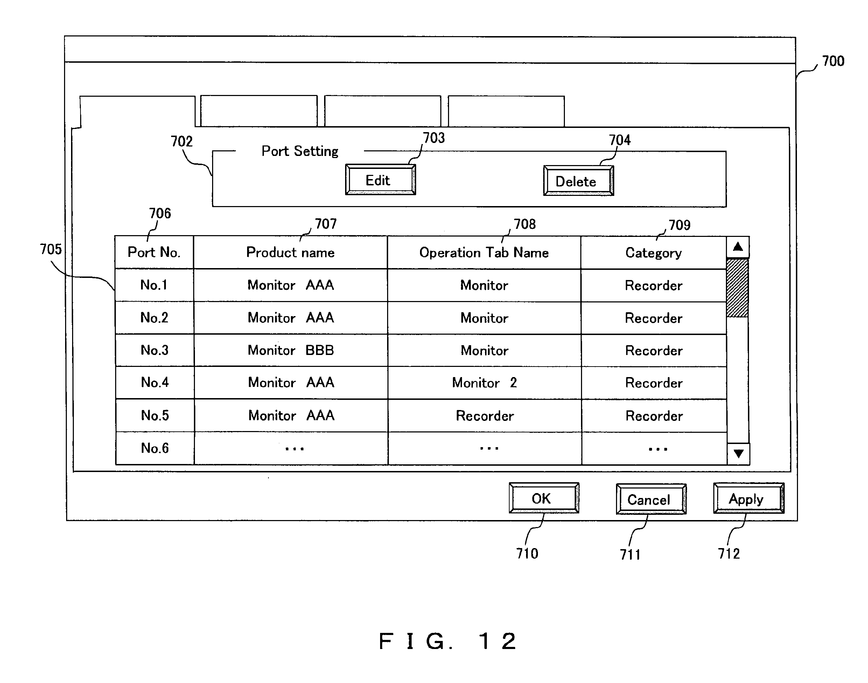

Fig. 12 shows a labeling window for a device connected to a communication port of the NMC 202 according to the present embodiment. A labeling window 700 can be used in the Administrator mode. In the labeling window 700, it is possible to set "Product name", "Operation Tab Name", and "Category" for each connection port of each connection device. The labeling window 700 includes a port setting area 702, a labeling setting list 705, an "OK" button 710, a "Cancel" button 711, and an "Apply" button 712.

The port setting area 702 has an "Edit" button 703 and a "Delete" button 704. By pressing the "Edit" button 703, it is possible to edit the labeling setting list 705. By pressing the "Delete" button 704, it is possible to delete an item selected arbitrarily in the labeling setting list 705.

The labeling setting list 705 includes items of "Port No." 706, "Product name" 707, "Operation Tab Name" 708, and "Category" 709. The "Port No." 706 is a number used for identifying the video signal input port or the video signal output port set in the video signal input/output unit 302. As the "Product name" 707, the name of the device (model name of the product) connected to the "Port No." is set. As the "Operation Tab Name" 708, the name that is labeled on the tab to be displayed on an operation window is set. As the "Category" 709, the category of the device is set.

When the "OK" button 710 is pressed, the window is closed after the labeling setting list information set in the labeling setting list 705 is fixed. When a "Cancel" button 711 is pressed, the labeling window 700 is closed. When an "Apply" button 712 is pressed, the information set in the labeling setting list 705 is reflected on the operation window. In this case, the labeling setting list information is stored in a labeling setting list table in the storage device 306.

When the same names as the "Operation Tab Name" 708 are to be set, the tabs having the same names are merged, and it is displayed as a common tab on the operation window. The merged tab is displayed as the second-layer tab. Then, the name labeled on second-layer tab has a numerical symbol that is added to the common name for each port in order to be distinguished from the other tabs.

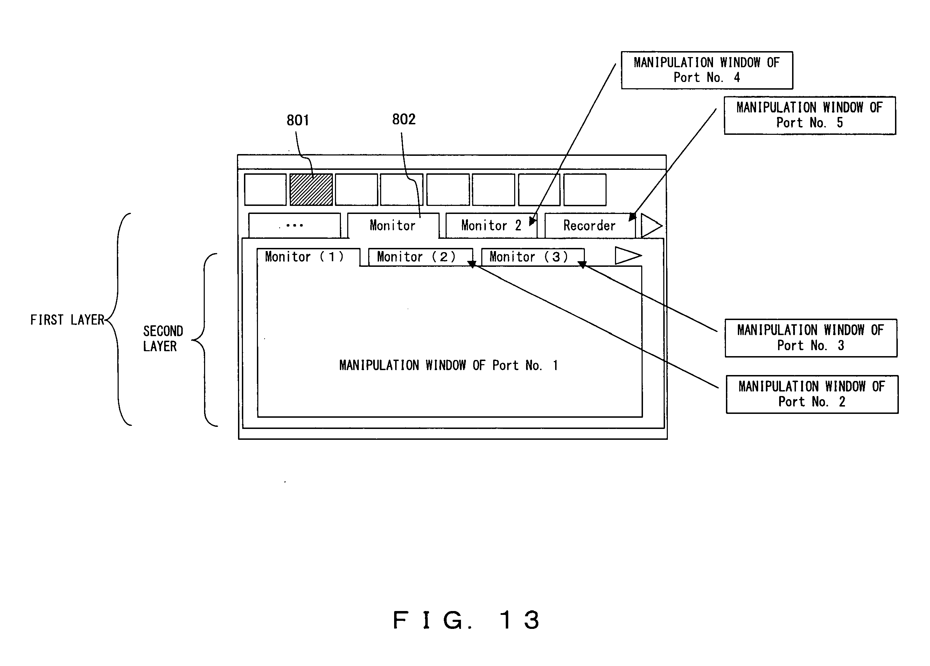

It is assumed that setting as shown in Fig. 12 is performed on the labeling setting list 705. Specifically, the categories of No. 1 through No. 5 are set to "Recorder". The "Operation Tab Name" 708 of "Port Number" No. 1 through No. 3 are set to "Monitor". The "Operation Tab Name" 708 of "Port Number" No. 4 and No. 5 are respectively set to "Monitor 2" and "Recorder". The operation window based on this setting is shown in Fig. 13.

Fig. 13 shows the operation window that was reformed on the basis of the setting content of the labeling window according to the present embodiment. When the category button "Recorder" 801 is pressed, first-layer tabs of "Monitor" 802, "Monitor 2" and "Recorder" are displayed. These are based on the setting of the "Category" 709 in the labeling setting list shown in Fig. 12.

When the "Monitor" 802 is selected, the second-layer tabs of the plurality of operation windows are displayed. This is based on the setting of "Operation Tab Name" 708 in the labeling setting list shown in Fig. 12.

As described above, when a device corresponding to a record that has the same name in the "Product name" 707 and that has the same category set as "Category" 709 is to be displayed on the operation window, a common tab is made and setting items can be displayed for a plurality of devices in one tab-content display area.

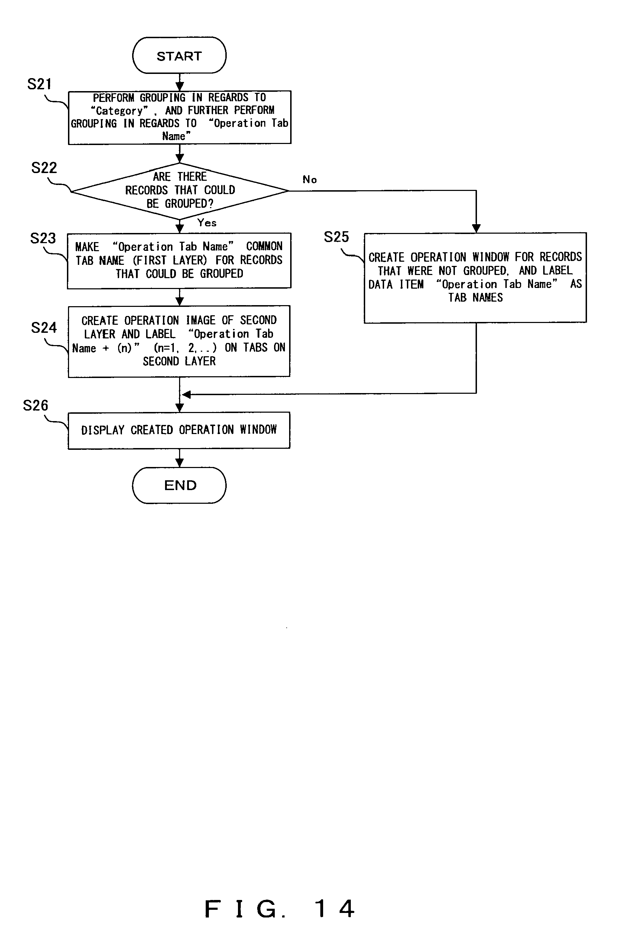

Fig. 14 is a flowchart for creating an operation window on the basis of the setting in the labeling setting list according to the present embodiment. In the labeling window 700, when the "Apply" button 712 is pressed after inputting the labeling setting list 705, the contents of the labeling setting list 705 are registered in the labeling setting list table in the storage device 306. The control unit 300 creates the operation window on the basis of the contents of the labeling setting list table.

First, the control unit 300 performs grouping on the basis of the data item "Category" in the labeling setting list table, and further performs grouping on the basis of the data item "Operation Tab Name" (S21). The records that were grouped undergo the process of S23 (Yes in S22). The records that could not be grouped undergo the process of S25 (No in S22).

The control unit 300 makes the names stored in the data item "Operation Tab Name" the common (first-layer) tab name (S23). The control unit 300 creates an operation window of the second layer, and labels "operation tab name+ (n) " (n=1, 2, ..) on the respective tabs in the second layer (S24). Thereafter, the operation window including the first layer and the second layer is displayed (S26) .

The control unit 300 creates an operation window for the records that were not grouped, and labels data item "Operation tab name" as tab names (S25). Thereafter, the operation window is displayed (S26).

As described above, even when a plurality of devices that are of the same kind are set in the labeling window 700, a tab is not added for each device, but a tab is added for each kind; accordingly, it is possible to suppress an increase in tabs in an operation window.

As described above, the medical support control system according to the present embodiment includes a first controller connected to at least one device, a second controller connected to at least one device, and a manipulation display device. The manipulation display device can alternately display a first graphical user interface (first GUI) created by the first controller and shared by the first and second controllers and a second graphical user interface (second GUI) created by the second controller (second GUI). The first and second GUIs respectively have at least switching devices and execution instruction parts. The switching part is a constituent element of GUIs for switching the first and second GUIs and for enabling control on the controller corresponding to the GUI after the switching. The execution instruction part is a constituent element of GUIs for giving instructions to execute processes in the respective controllers.

Thereby, it is possible to control the two controllers as one controller. Accordingly, it is possible to reduce the sense of discomfort caused when the controllers are switched during the manipulation on the TP.

According to the present embodiment, each one of the two controllers receives from the other controller information that controls the other controller, and can create the switching part for itself on the basis of the received information. Thereby, the two controllers have manipulation windows similar to each other, and accordingly it is possible to reduce the sense of discomfort caused when the controllers are switched during the manipulation on the TP.

Also, according to the present embodiment, when the first and second GUIs are switched on the basis of the switching part, it is possible to display an intermediate image until the switching is completed. This intermediate image includes at least one common part (such as a category button, a tab, or the like) among the parts that are shared by the first and second GUIs.

Thereby, when a window after switching is to be displayed due to the switching of the tabs and also the target controller is to be switched, the intermediate image can be displayed temporarily until the window after the switching becomes ready to be displayed.

According to the present embodiment, the switching part includes a category selection part and a sub-category selection part. The category selection part is a constituent element (category button) of GUIs for selecting a category in which menus in the first layer for selecting functions available to the first and second controllers are categorized. The sub-category selection part is a constituent element (tab) of GUIs for selecting a sub category in which menus on the second layer existing under the respective categories are categorized. Sub-category information representing the state of the sub category in a category before being selected by the category selection part is held for each category. This sub-category information is shared by the controllers. Accordingly, when one of the categories is selected, the state of the sub category can be reproduced on the basis of the sub-category information.

Thereby, when categories are switched when the category button is pressed, users feel as if they have controlled only one controller and do not feel a sense of discomfort even during the switching of the controllers.

According to the present embodiment, by the manipulation display device, it is possible to set which of the categories categorized for selecting functions available to the first and second controllers the respective devices are to belong, and to set the names for labeling for the sub categories in order to categorize the devices at a lower level under the respective categories. Thereby, among the set devices, for devices whose category names are the same and whose names labeled on the sub categories are the same, manipulation items of the devices are merged in the same sub category and can be displayed in the manipulation display device.

Thereby, even when a plurality of devices that are of the same kind are set in the labeling window, it is possible to suppress an increase in tabs in the operation window because a tab is not added for each device, but is added for each kind.

### <Second Embodiment>

Described in the second embodiment is a medical support control device to which are connected a display manipulation device and a medical device control device connected to a medical device and controlling the medical device, comprising: a detection unit for detecting abnormality in an image signal line outputting an image signal to the display manipulation unit; and an interruption unit for interrupting communications to the medical device control device in accordance with the detection unit. The configuration of the system of the second embodiment is the same as the configuration of the system shown in Figs. 1 to 3. Note that the same component sign is assigned to the same configuration as the first embodiment and the description is omitted in the description of the present embodiment.

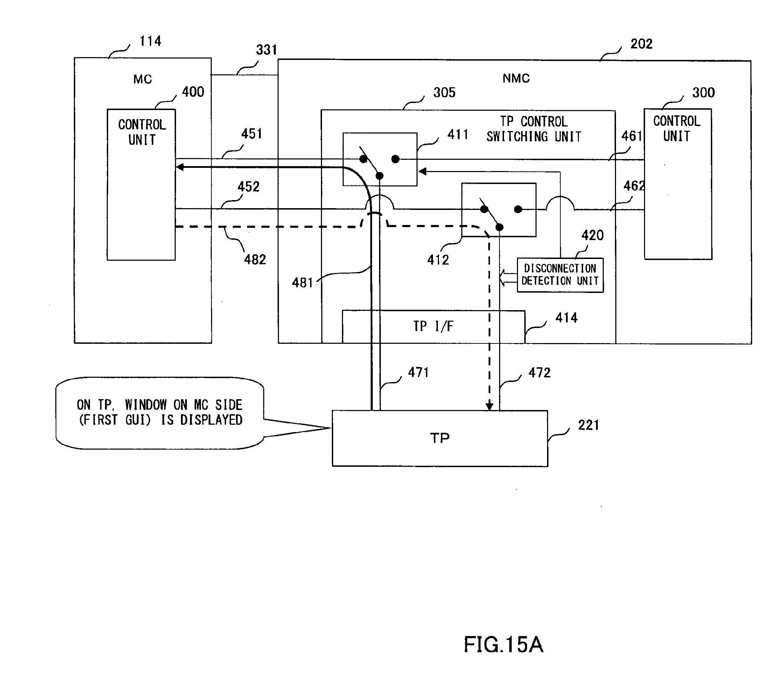

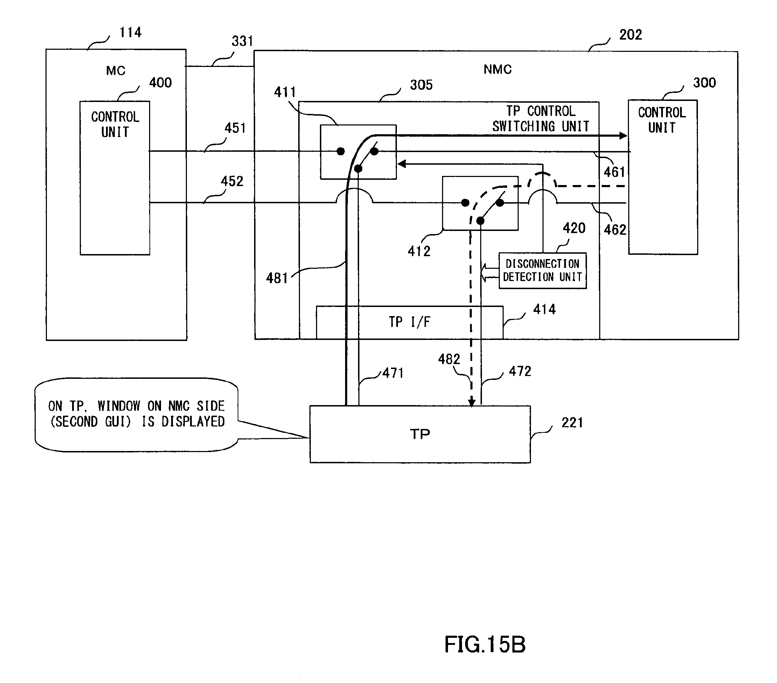

Figs. 15A and 15B respectively show the switching of the control targets between the NMC 202 and the MC 114 by using the TP control switching unit 305.

As described above, the MC 114 is a controller mainly for medical devices. The MC 114 creates a graphical user interface (first GUI) that is a window used for controlling the medical devices belonging to the MC 114 itself.

As described above, the NMC 202 is a controller mainly for non-medical devices. The NMC 202 creates a graphical user interface (second GUI) that is a window used for controlling the non-medical devices belonging to the NMC 202 itself.

The first and second GUIs are designed on the basis of a common graphical user interface (common GUI). For example, by switching the tabs on a window on the TP, the first and second GUIs can be switched therebetween.

The TP coordinate communication lines 451, 461, and 471 are communication lines used for transmitting the TP coordinate signals of the TP 221 to a control unit 400 of the MC 114 or to the control unit 300 of the NMC 202.

The TP image lines 452, 462, and 472 are image lines for transmitting images such as the first or second GUI to be displayed on the TP 221 from the control unit 400 of the MC 114 to the TP 221 or from the control unit 300 of the NMC 202 to the TP 221.

The TP control switching unit 305 has a TP coordinate communication line switch 411, a TP image line switch 412, a disconnection detection unit 420, and a TP I/F 414.

The TP I/F 414 is an interface to which the TP image line and the TP coordinate communication lines used for connecting the TP 221 and the NMC 202 are connected.

The TP coordinate communication line switch 411 is used for determining whether the TP coordinate communication line 471 is to be connected to the TP coordinate communication line 451 on the MC 114 or to the TP coordinate communication line 461 on the NMC 202 side.

The TP image line switch 412 is used for determining whether the TP image line 472 is to be connected to the TP image line 452 on the MC 114 side or to the TP image line 462 on the NMC 202 side.

The disconnection detection unit 420 detects disconnection of the TP image line 472 by detecting the states of voltage being applied to the TP image line 472 or the voltage value in the TP image line 472. The disconnection detection unit 420 reports the detection results to the TP coordinate communication line switch 411.

Explanations will be given for a case, shown in Fig. 15A, in which the TP coordinate communication line 451 is selected by the TP coordinate communication line switch 411 and the TP image line 452 is selected by the TP image line switch 412. A TP image signal 482 generated in the control unit 400 is input into the TP 221 via the TP image line switch 412, and the first GUI is displayed on the TP 221. In this case, a TP coordinate signal 481 that is generated by the touch manipulations on the first GUI displayed on the TP 221 is input from the TP 221 to the control unit 400 via the TP coordinate communication line switch 411.

Next, explanations will be given for a case, shown in Fig. 15B, in which the TP coordinate communication line 461 is selected by the TP coordinate communication line switch 411, and the TP image line 462 is selected by the TP image line switch 412. The TP image signal 482 generated in the control unit 300 is input into the TP 221 via the TP image line switch 412, and the second GUI is displayed on the TP 221. The TP coordinate signal 481 that is generated by touch manipulations of the second GUI displayed on the TP 221 is input from the TP 221 to the control unit 300 via the TP coordinate communication line switch 411.

Next, explanations will be given for a case in which the first GUI transitions to the second GUI in response to the touch manipulations on the TP 221. The TP coordinate signal 481 generated by the touch manipulations on the TP 221 is sent to the control unit 400 in the MC 114. The control unit 400 that has received the TP coordinate signal 481 reports, to the control unit 300 in the NMC 202, that the first GUI will be switched to the second GUI. When receiving this report, the control unit 300 controls the TP control switching unit 305, and the switches 411 and 412 are operated so that the TP coordinate communication line 461 and the TP image line 462 on the NMC side are enabled. Then, the control unit 300 causes the TP 221 to display the second GUI via the TP image lines 462 and 472. Additionally, this process is also applied to the case in which the second GUI transitions to the first GUI in response to the touch manipulations.

The control unit 300 and the control unit 400 communicate with each other via the communication line 331 to monitor each other. Also, the control unit 300 and the control unit 400 synchronize with each other's GUI environment, and exchange information that has to be held by both of them for configuring a common GUI. An example of the above information is window elements (window element information such as tab names) to be used commonly.

As described above, the target to be controlled by the TP 221 is changed between the MC 114 and the NMC 202 on the basis of the switching operations of the TP control switching unit 305. This switching operation is not perceived by the users, and accordingly the users feel as if they have controlled only one controller.

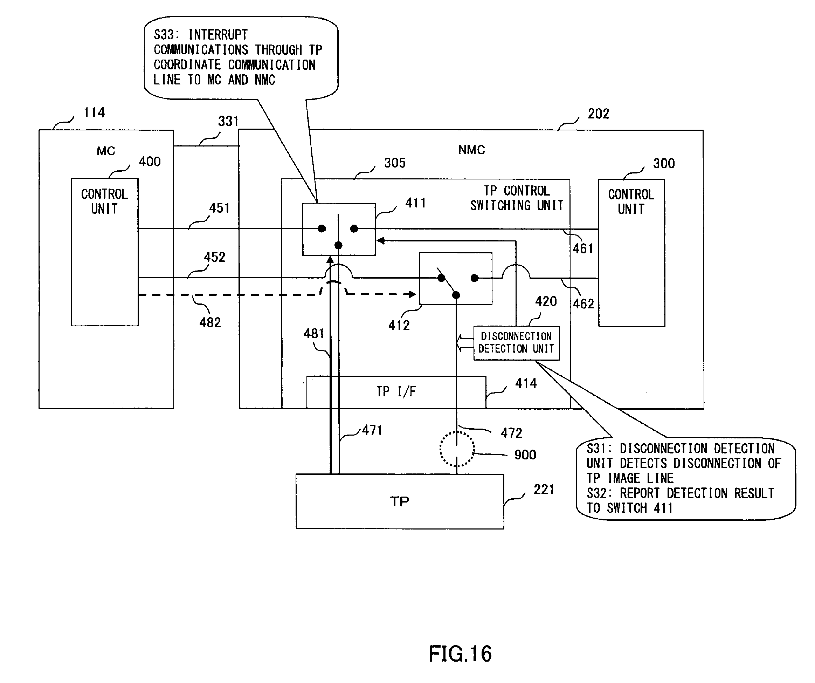

Fig. 16 shows, for the present embodiment, the interruption of communications through the TP coordinate communication line to the MC 114 and the NMC 202 when the TP image line is disconnected. For example, it is assumed that the TP image line 472 is disconnected, as pointed out by numeral 900 in the case of Fig. 16. In this case, the disconnection detection unit 420 detects the disconnection of the TP image line (S31) , and reports this result to the TP coordinate communication line switch 411 (S32).

The switch 411, having received this report, interrupts the communications through the TP coordinate communication line to the MC 114 and the NMC 202 (S33). In other words, the switch 411 does not connect the TP coordinate communication line 471 to either the TP coordinate communication line 451 on the MC 114 side or the TP coordinate communication line 461 on the NMC 202 side.

As described above, the NMC 202 includes a detection unit (the disconnection detection unit 420) for detecting the abnormality of the image signal line used for outputting image signals to the TP 221 and an interruption unit (the switch 411) for interrupting communications with the medical device control device (MC 114). The interruption unit also interrupts the communications with the NMC 202 itself.

By the above configuration, when the TP image line 472 is disconnected, it is possible to interrupt the communications through the TP coordinate communication line. Accordingly, when the TP screen is blacked out due to the disconnection in the TP image line 472, it is impossible to control the MC 114 or the NMC 202 through the TP 221. Thus, when the screen of the TP 221 is blacked out due to the disconnection in the TP image line, it is possible to prohibit the manipulations of the medical devices connected to the MC 114 and the NMC 202.

The scope of the present invention is not limited to any of the above embodiments, and various other configurations and embodiments are allowed without departing from the spirit of the present invention.

As described above, it is possible to provide a medical support control device for controlling medical devices and non-medical devices.

## Claims

1. A medical support control system comprising:
a first controller (114, 202) connected to at least one device;
a second controller (202, 114) connected to at least one device; and
a manipulation display device (221) shared by the first controller and the second controller, and alternately displaying a first graphical user interface (first GUI) created by the first controller and a second graphical user interface (second GUI) created by the second controller, wherein:
each of the first GUI and the second GUI includes at least:
a switching part (502-509, 511) for performing switching between the first GUI and the second GUI, and enabling control on a controller corresponding to a GUI after the switching; and
an execution instruction part (521-525) for instructing the respective controllers to execute a process.

2. The medical support control system according to claim 1, wherein:
each of the controllers (114,202) receives information for controlling the other controller from said other controller, and creates the switching part of said each controller itself on the basis of the received information.

3. The medical support control system according to claim 1, wherein:
when the first GUI and the second GUI are switched on the basis of the switching part, an intermediate image (553) is displayed until the switching is completed.

4. The medical support control system according to claim 1, wherein:
the intermediate image (553) has at least one common part.

5. The medical support control system according to claim 1, wherein:
the switching part:
comprises:
a category selection part (502-509) for selecting a category in which menus on a first layer for selecting functions available to the first and second controllers are categorized; and
a sub-category selection part for selecting a sub category in which menus on a second layer existing under the respective categories are categorized; and
holds, for each category, sub-category information representing a state of the sub category in a category before being selected by the category selection part;
shares the sub-category information between the controllers; and
reproduces the state of the sub category on the basis of the sub-category information when one of the categories is selected (S11-S14).

6. The medical support control system according to claim 1, wherein:
by the manipulation display device, which of the categories categorized for selecting functions available to the first and second controllers and a name for labeling on a sub category for making a category be able to be categorized at a lower level of the respective categories are set as the second GUI for each device (S23); and
among the set devices, for devices whose category names are the same and whose names labeled on the sub categories are the same, manipulation items of the devices are merged in the same sub category and are displayed in the manipulation display device (S24).
